# EUROPÄISCHE PATENTSCHRIFT

(11) **EP 1 441 678 B1**
(45) Veröffentlichungstag und Bekanntmachung des Hinweises auf die Patenterteilung: **13.06.2007**
(21) Anmeldenummer: 02787508.7
(22) Anmeldetag: 25.10.2002
(51) Int. Cl.: A61F 9/013

(54) **SCHNEIDVORRICHTUNG**
CUTTING DEVICE
DISPOSITIF DE COUPE

(30) Priorität: 25.10.2001 DE 10152152
(43) Veröffentlichungstag der Anmeldung: 04.08.2004
(73) Patentinhaber: Carl Zeiss Meditec AG, 07745 Jena (DE)
(72) Erfinder: DICK, Manfred, 07926 Gefell (DE); SCHRÖDER, Eckhard, 90542 Eckental (DE); WILKE, Martina, 99099 Erfurt (DE)
(74) Vertreter: DTS München
(86) Internationale Anmeldenummer: PCT/EP2002/011926
(87) Internationale Veröffentlichungsnummer: WO 2003/034958

(56) Entgegenhaltungen:
- WO-A-01/05341
- WO-A-01/19303
- DE-A- 10 119 477
- DE-C- 3 409 798
- US-A- 5 269 795

## Beschreibung

Die vorliegende Erfindung betrifft eine Schneidvorrichtung zur Entnahme eines Epithelflaps von einem Auge sowie ein Verfahren zur Entnahme eines Epithelflaps von einem Auge.

In der Ophthalmologie ist es bekannt, die Hornhaut bei Sehschwäche durch Ablation von Gewebe zu formen. Bewährt hat sich hierfür die Laserstrahlung eines ARF-Eximerlasers, dessen gepulste Strahlung eine Wellenlänge von 193 nm hat. Mit Strahlung dieser Wellenlänge werden gute Abtragungsergebnisse bei geringsten vernachlässigbaren Nebenwirkungen erzielt. Dabei unterscheidet man zwei Verfahren zur Durchführung dieser Operation:

Bei der fotorefraktiven Keratektomie (PRK) wird die obere etwa 50 Mikrometer dicke Epithelschicht von der Bowmannschen Membran mit diversen Hockeymessern irreversibel entfernt und die Laserablation auf der stromalen Oberfläche durchgeführt. Innerhalb eines Heilungsprozesses bildet sich nach der Operation neues Epithel auf der laserbehandelten Oberfläche. Das Verfahren ist jedoch mit Schmerzen für den Patienten verbunden.

Bei der lasergestützten intrastromalen Keratomileusis (LASIK) wird mit Hilfe eines Mikrokeratoms ein stromaler Lappen (Flap) mit einer Dicke von ca. 160 Mikrometern von der Hornhaut gelöst und zurückgeklappt. Die Laserbehandlung wird im intrastromalen Gewebe durchgeführt und nach der Behandlung der Flap zurückgeklappt. Dabei haben die Patienten geringste Schmerzen und eine schnelle Visiuserholung nach der Operation. Der Eingriff mit einem Mikrokeratom ist jedoch riskobehaftet und die zur refraktiven Korrektur verfügbare Restdicke der Hornhaut ist geringer als bei der zuvor dargestellten PRK.

Die WO 01 053 41 beschreibt eine Vorrichtung zum Einbringen eines ringförmigen Schnittes in die Cornea parallel zur Corneaachse, wobei die Vorrichtung einen Aufbau und Mittel zum Applizieren des Aufbaus auf die vordere Oberfläche einer Cornea aufweist.

Neuerdings wird deshalb ein neues Verfahren betrachtet, bei dem man einen Epithellappen prepariert, der vor der PRK beiseite geschoben wird, um nachher wieder reproduzierbar das offene Stroma abzudecken und anzuheilen. Diese Methode der lasergestützten subepithelialen Keratomileusis (LASEK) vermeidet einerseits das chirurgische Risiko des Mikrokeratomes sowie der größeren Schwächung der Hornhaut bei dem zuvor gestellten LASIK-Verfahren, bietet jedoch dem Patienten die gleiche schmerzarme Heilung wie bei LASIK (siehe dazu "LASEK: Notes from the new fronsher" Aufsatz von Daniel S. Durrie, veröffentlicht in (?)).

Die Preparation des Epithellappens bei LASEK ist jedoch momentan noch eine dem Geschick des Arztes überlassene Angelegenheit, der das vorher mit Alkohol angelöste Epithel mit mehr oder weniger Erfolg und einfachen chirurgischen Instrumenten zur Seite schiebt, um es anschließend wieder passgerecht zu platzieren. Im Fehlerfall wird so oft aus LASEK eine PRK. Außerdem führt die Benutzung von Alkohol ebenfalls zur Schädigung des Epithels oder auch des stromalen Gewebes, wenn die Einwirkung zu lange geschieht und deshalb auch tiefer erfolgt.

Es ist daher Aufgabe der vorliegenden Erfindung, ein Verfahren und eine Vorrichtung bereitzustellen, die eine einfache und sichere Entnahme eines Epithelflaps ermöglicht.

Dieses Problem wird durch eine Schneidvorrichtung zur Entnahme eines Epithelflaps von einem Auge mit einem im Wesentlichen rotationssymmetrischen Schneidenkörper mit einer Schnittkante und einem Freiraum mit einer Anlagefläche für das Epithelflap sowie eine Einrichtung zur Fixierung des Epithelflaps an der Anlagefläche nach Anspruch 1 sowie ein Verfahren zur Entnahme eines Epithelflaps von einem Auge, welches die Schritte Einbringen eines Schnittes mit einer Schneidvorrichtung in das Epithel bis etwa auf Tiefe der Bowannschen Membran, Fixierung des Epithelflaps an der Schneidvorrichtung, Entnahme des Epithelflaps mit der Schneidvorrichtung gemäß Anspruch 12 umfasst, gelöst.

Besonders bevorzugt kann durch diese Schneidvorrichtung ein "Schnitt" dadurch durchgeführt werden, dass die Schneidvorrichtung mit der Anlagefläche für das Epithelflap auf das Auge bzw. das Epithel aufgesetzt wird und der mit der Schneidvorrichtung in Berührung stehende Teil des Epithels als Epithelflap an der Schneidvorrichtung anhaftet. Das Epithelflap kann nun vom Auge dadurch entfernt werden, dass die Schneidvorrichtung vom Auge wegbewegt wird und das Epithelflap an der Schneidvorrichtung haften bleibt, mithin "vom Auge abgerissen" wird. Ganz besonders bevorzugt wird so ein gekühlter Stempel als Schneidvorrichtung auf das Epithel aufgebracht, so dass das Epithel am Stempel anfriert. In einem zweiten Schritt wird der Stempel entfernt, wobei das Epithel mit entfernt wird. An die Entfernung des Epithels schließt sich die Ablation des Gewebes zur Korrektur der Fehlsichtigkeit an. Nach erfolgter Operation kann das Epithel wieder aufgebracht werden, wobei die Trennung des Epithels vom Stempel vorzugsweise durch Erwärmung des Stempels erfolgt. Zum Schutz während der Phase der Heilung kann eine therapeutische Kontaktlinse (vorzugsweise Brechkraft 0-Dioptrien) dienen. Durch Wahl der Temperatur und der Oberflächenbeschaffenheit der Schneidvorrichtung kann so auch die Dicke des abzulösenden Epithelflaps optimal vorbestimmt werden. Bevorzugte Materialen der mit dem Epithel in Berührung kommenden Fläche der Schneidvorrichtung sind biokompatible Materialien.

In einer Weiterbildung der erfindungsgemäßen Schneidvorrichtung umfasst die Einrichtung zur Fixierung des Epithelflaps eine Ansaugvorrichtung, die einen Unterdruck zwischen dem Freiraum und dem Epithelflap erzeugen kann. Mit Hilfe des erzeugten Unterdruckes wird das Epithelflap sicher an die Anlagefläche herangezogen und an dieser vorübergehend festgelegt, so dass das Epithelflap entnommen werden kann.

Alternativ ist die Einrichtung zur Fixierung des Epithelflaps ein Kühlelement. Mit dem Kühlelement kann die Anlagefläche soweit heruntergekühlt werden, dass das Epithelflap an diesem anfriert. Das Ablösen des Epithelflaps geschieht durch Erwärmen der Anlagefläche, beispielsweise durch aktive Wärmezufuhr oder durch schlichtes Ausschalten des Kühlelementes und einer Erwärmung auf Umgebungstemperatur.

In einer Weiterbildung der Schneidvorrichtung ist das Kühlelement ein Peltierelement. Es handelt sich hierbei um gängige Elemente zur Kühlung, die entsprechend sicher und kostengünstig einsetzbar sind. Alternativ kann das Kühlelement ein Kryoelement sein.

Vorteilhaft ist es, wenn die Anlagefläche gegenüber dem Schneidenkörper drehbar ist. Das durch Unterdruck bzw. Anfrieren an der Anlagefläche vorübergehend festgelegte Epithelflap kann durch die Drehbewegung der Anlagefläche von dem restlichen Eptihel abgeschert werden.

Ebenso ist es vorteilhaft, wenn die Anlagefläche gegenüber dem Schneidenkörper radial verschiebbar ist. Das zuvor beschriebene Abscheren des Epithelflaps von dem restlichen Epithel kann so durch eine translatorische Bewegung erfolgen.

In einer Weiterbildung der Schneidvorrichtung umfasst diese zusätzlich eine Schneideinheit, die das Epithelflap vollständig von dem Auge abtrennen kann. Die Abtrennung des Epithelflaps erfolgt dabei durch eine Schneidbewegung, so dass eine sehr genaue Dicke des Epithelflaps erzielbar ist.

Die Schneideinheit umfasst vorteilhaft einen Draht. Dieser fungiert als messerartige Schneide und wird durch das abzulösende Epithel geführt, indem der Schneiddraht an der Schnittkante der Schneidvorrichtung vorbeigeführt wird.

Vorteilhaft umfasst die Schneidvorrichtung einen Schlitten, wobei der Draht entlang des Schlittens bewegbar ist. Mit dieser Maßnahme kann die Schneidvorrichtung vergleichsweise einfach ausgeführt werden.

Eine genaue Positionierung der Schneidvorrichtung auf dem zu behandelnden Auge wird ermöglicht, wenn die Schneidvorrichtung einen Saugring zur Fixierung der Schneidvorrichtung auf dem Auge umfasst.

In einer Weiterbildung des erfindungsgemäßen Verfahrens ist vorgesehen, dass das Epithelflap mit einer Schneideinheit vollständig von dem Auge abgetrennt wird.

Alternativ kann das Epithelflap durch eine Drehung oder Verschiebung von dem Auge abgeschert werden. Beide zuvor genannten Maßnahmen ermöglichen die Entnahme eines in seiner Dicke genau definierten Epithelflaps.

In einer Weiterbildung des erfindungsgemäßen Verfahrens ist vorgesehen, dass das Epithelflap an einer Kontaktlinse festgelegt wird. Diese Maßnahme bietet den Vorteil, dass das entnommene Epithelflap mittels der Kontaktlinse nach Ende der Laserbehandlung wieder appliziert werden kann. Die Kontaktlinse ermöglicht eine einfache Handhabung des Epithelflaps und schützt dieses während des Heilungsprozesses.

Vorteilhaft wird das Epithelflap an der Kontaktlinse mittels eines Klebstoffes angeklebt. Auf diese Weise kann auf eine mechanische Fixierung verzichtet werden.

Vorteilhaft ist, wenn der Klebstoff sich unter Einfluß von Körperwärme und/oder Tränenflüssigkeit auflöst. Nach dem Aufbringen des Epithelflaps mittels der Kontaktlinse löst sich die Klebverbindung so selbständig auf und die Kontaktlinse kann wieder entfernt werden.

Der Klebstoff kann vorteilhaft ein Fibrinklebstoff sein. Es handelt sich dabei um einen handelsüblichenklebstoff, der sich unter Einfluß von Körperwärme und/oder Tränenflüssigkeit auflöst.

Im Folgenden wird ein Ausführungsbeispiel der vorliegenden Erfindung anhand der beiliegenden Zeichnungen näher erläutert. Dabei zeigen:
- Fig. 1: eine erste Ausführungsform einer erfindungsgemäßen Schneidvorrichtung im Schnitt;
- Fig. 2: eine zweite Ausführungsform einer erfindungsgemäßen Schneidvorrichtung im Schnitt;
- Fig. 3: eine dritte Ausführungsform einer erfindungsgemäßen Schneidvorrichtung im Schnitt;
- Fig. 4: eine vierte Ausführungsform einer erfindungsgemäßen Schneidvorrichtung in der Seitenansicht;
- Fig. 5: die vierte Ausführungsform gemäß Fig. 4 in der Draufsicht.

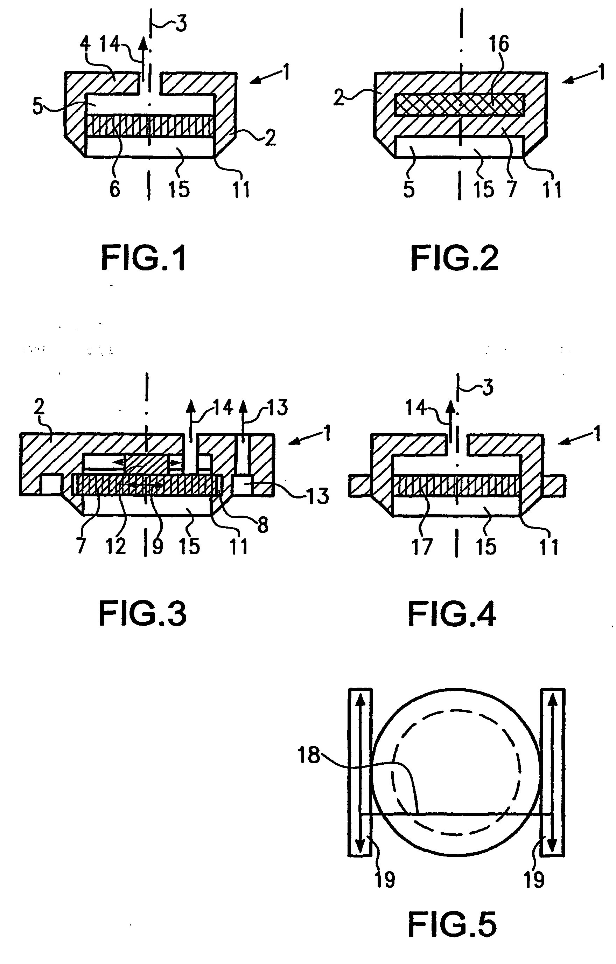

Zunächst wird auf Fig. 1 Bezug genommen. Dargestellt ist eine erste Ausführungsform einer Schneidvorrichtung 1. Diese umfasst einen rotationssymmetrischen Schneidenkörper 2, dessen Rotationsachse in der Fig. 1 mit dem Bezugszeichen 3 versehen ist. Der Einfachheit halber ist nur der Schneidenteil dargestellt, nicht dargestellt sind die für den Antrieb und die Halterung der Schneidvorrichtung 1 notwendigen weiteren Bestandteile. Die Schneidvorrichtung 1 kann beispielsweise in einer Aufnahmevorrichtung ähnlich einem Bohrfutter oder dergleichen aufgenommen werden. Dazu verfügt die Schneidvorrichtung 1 über eine Aufnahme 4, die hier der Einfachheit halber nicht näher dargestellt ist. Der Schneidenkörper 2 ist insgesamt becherförmig und verfügt im Bereich der Aufnahme 4 über eine Ansaugvorrichtung 14, mit der ein Unterdruck in einem Freiraum 5 erzeugt werden kann. Auf der der Aufnahme 4 gegenüber liegenden Seite verfügt die Schneidvorrichtung 1 über eine Schnittkante 11. Ein Saugring 13 ermöglicht die Fixierung der Schneidvorrichtung an dem zu behandelnden Auge, indem durch Erzeugung eines Unterdruckes in dem Saugring 13 die gesamte Schneidvorrichtung an das Auge angesaugt wird.

Bei einem Betrieb der Schneidvorrichtung 1 wird diese um die Rotationsachse 3 in Rotation versetzt. Die Schnittkante 11 wird in das Epithel eingefahren bis auf die Bowmannsche Membran. Durch Erzeugen eines Unterdruckes im Freiraum 5 mit Hilfe der Ansaugvorrichtung 14 wird ein Epithelflap 6 von dem Auge abgehoben.

Fig. 2 zeigt eine weitere Ausführungsform einer Schneidvorrichtung 1. Im Unterschied zu der in Fig. 1 dargestellten Ausführungsform wird hier das Epithelflap 6 nicht durch ein Unterdruck in einen Freiraum 5 eingesogen, sondern an einer Anlagefläche 7 angefroren. Dazu verfügt die Schneidvorrichtung 1 über ein Peltier- bzw. Kryoelement 16. Dieses ist an der Anlagefläche 7 angeordnet und kann die Anlagefläche 7 auf eine Temperatur unterhalb des Gefrierpunktes von Wasser oder einer in den Freiraum 5 einzubringenden Flüssigkeit kühlen.

Die Anwendung dieser zweiten Ausführungsform einer Schneidvorrichtung 1 erfolgt sinngemäß wie die der anhand der Fig. 1 dargestellten Ausführungsform. Die Schneidvorrichtung 1 wird in Rotation versetzt und die Schnittkante 11 in das Epithel eingefahren. Bei einem genügend tiefen Einschnitt wird mittels des Peltier- oder Kryoelementes 16 das abgetrennte Epithelflap 6 an der Anlagefläche 7 angefroren und kann somit abgehoben werden.

In Fig. 3 ist eine weitere Ausführungsform einer erfindungsgemäßen Schneidvorrichtung dargestellt. Auch diese umfasst einen Schneidenkörper 2. Im äußeren Bereich des Schneidenkörpers 2 ist ein Saugring 13 zur Fixierung auf dem Auge angeordnet. Oberhalb der Anlagefläche 7, die den Freiraum 5 begrenzt, ist ein Schwingelement 12 angeordnet. Dieses kann ein Peltierelement 16 mit poröser oder gelöcherter Grundplatte 17 sein. Die Anlagefläche 7 ist radialbeweglich in einer Ringnut 8 des Schneidenkörpers 2 angeordnet. Das Schwingelement 12 stützt sich mit einer Seite an den Schneidenkörper 2 und mit einer anderen Seite an der Anlagefläche 7 ab. Wird das Schwingelement betätigt, so erfolgt eine translatorische Bewegung der Anlagefläche 7, dies ist durch einen Doppelpfeil 9 in Fig. 3 angeordnet. Die Anlagefläche 7 der Grundplatte 17 ist porös oder weist eine Vielzahl von Durchgangsbohrungen auf, so dass mittels der Ansaugvorrichtung 14 aus dem Freiraum 5 für das Epithelflap 6 Luft abgesaugt werden kann und das Epithelflap 6 somit an die Anlagefläche 7 angesaugt wird.

Bei der Anwendung der Vorrichtung nach Fig. 3 wird wie zuvor dargestellt vorgegangen, zusätzlich wird das Ablösen des Epithelflaps durch eine Rüttelbewegung der Anlagefläche 7 erleichtert.

Fig. 4 und 5 zeigen eine Erweiterung der Schneidvorrichtung 1. Dabei ist in Fig. 4 ein Axialschnitt dargestellt, in Fig. 5 ist eine Prinzipskizze der Draufsicht dargestellt. Die zuvor beschriebenen Ausführungsformen der Schneidvorrichtung 1 sind hier ergänzt um eine weitere Schneideinheit 15. Diese umfasst hier einen dünnen Draht 18, der mit Hilfe eines Schlittens 19 als Führung an der Schnittkante 11 für das Epithelflap 6 vorbeigeführt werden kann. Auf diese Art und Weise wird das Epithelflap 6 sicher von dem restlichen Epithel abgetrennt. Eine Halterung des Epithels kann wie bei den zuvor dargestellten Vorrichtungen beispielsweise durch Unterdruck oder durch Anfrieren erfolgen.

Alternativ kann die Halterung des Epithelflaps 6 durch Haftung an einer weichen Kontaktlinse erfolgen, wobei die Haftung dadurch erreicht wird, dass jeweils eine Komponente eines handelsüblichen Fibrinklebers oder einer anderen körpereigenen, optisch klaren und/oder sich unter Einfluss von Körperwärme bzw. Tränenflüssigkeit definiert selbstauflösenden Substanz an der Kontaktlinse und die andere Komponente auf das Epithel appliziert wird, was zu einer temporären und reversiblen Verschmelzung des Epitheles mit dem Fibrinkleber führt und/oder eine klebende Substanz allein auf der Kontaktlinse enthalten ist, was nach Markierung der zu lösenden Epithelfläche zu einer kompletten Ablösung des Epithels und Haftung an der Linse führt. Zur Repositionierung und zur Anheilung des Epithellappens wird die Kontaklinse temporär auf dem laserbehandelten Auge belassen.

| | |
|---|---|
| BEZUGSZEICHENLISTE | |
| 1 | Schneidvorrichtung |
| 2 | Schneidenkörper |
| 3 | Rotationsachse |
| 4 | Aufnahme |
| 5 | Freiraum |
| 6 | Epithelflap |
| 7 | Anlagefläche |
| 8 | Ringnut |
| 9 | Doppelpfeil |
| 11 | Schnittkante |
| 12 | Schwingelement |
| 13 | Saugring |
| 14 | Ansaugvorrichtung |
| 15 | Schneideinheit |
| 16 | Peltier- oder Kryoelementes |
| 17 | Grundplatte |
| 18 | Draht |
| 19 | Schlitten |

## Patentansprüche

1. Schneidvorrichtung (1) zur Entnahme eines Epithelflaps (6) von einem Auge mit einem im wesentlichen rotationssymmetrischen Schneidenkörper (2) mit einer Schnittkante (11) und mit einer Einrichtung zur Fixierung des Epithelflaps (6) an einer Anlagefläche (7), **dadurch gekennzeichnet dass** die Schneidvorrichtung weiter einen Freiraum (15) für das Epithelflap (6) aufweist.

2. Schneidvorrichtung (1) nach Anspruch 1, bei der die Einrichtung zur Fixierung des Epithelflaps (6) eine Ansaugvorrichtung (14) umfasst, die einen Unterdruck zwischen dem Freiraum (15) und dem Epithelflap (6) erzeugen kann.

3. Schneidvorrichtung (1) nach Anspruch 1, bei der die Einrichtung zur Fixierung des Epithelflaps (6) ein Kühlelement (16) umfasst.

4. Schneidvorrichtung (1) nach Anspruch 3, bei der das Kühlelement (16) ein Peltierelement ist.

5. Schneidvorrichtung (1) nach Anspruch 3, bei der das Kühlelement (16) ein Kryoelement ist.

6. Schneidvorrichtung (1) nach einem der vorhergehenden Ansprüche, bei der die Anlagefläche (7) gegenüber dem Schneidenkörper (2) drehbar ist.

7. Schneidvorrichtung (1) nach einem der vorhergehenden Ansprüche, bei der die Anlagefläche (7) gegenüber dem Schneidenkörper (2) radial verschiebbar ist.

8. Schneidvorrichtung (1) nach einem der vorhergehenden Ansprüche, wobei diese zusätzlich eine Schneideinheit 15 umfasst, die das Epithelflap (6) vollständig von dem Auge abtrennen kann.

9. Schneidvorrichtung (1) nach Anspruch 8, bei der die Schneideinheit einen Draht (18) umfasst.

10. Schneidvorrichtung (1) nach Anspruch 9 mit einem Schlitten (9), wobei der Draht (18) entlang des Schlittens (9) bewegbar ist.

11. Schneidvorrichtung (1) nach einem der vorhergehenden Ansprüche, wobei diese einen Saugring (13) zur Fixierung der Schneidvorrichtung (1) auf dem Auge umfasst.

## Claims

1. Cutting device (1) for the removal of an epithelial flap (6) from an eye with an essentially rotation-symmetrical cutting element (2) with a cutting edge (11) and with an apparatus for fixing the epithelial flap (6) to a bearing surface (7), **characterized in that** the cutting device also has a free space (15) for the epithelial flap (6).

2. Cutting device (1) according to claim 1, in which the apparatus for fixing the epithelial flap (6) comprises a suction device (14) that can produce a negative pressure between the free space (15) and the epithelial flap (6).

3. Cutting device (1) according to claim 1, in which the apparatus for fixing the epithelial flap (6) comprises a cooling element (16).

4. Cutting device (1) according to claim 3, in which the cooling element (16) is a Peltier element.

5. Cutting device (1) according to claim 3, in which the cooling element (16) is a cryogenic element.

6. Cutting device (1) according to one of the previous claims, in which the bearing surface (7) is rotatable vis-à-vis the cutting element (2).

7. Cutting device (1) according to one of the previous claims, in which the bearing surface (7) is radially displaceable vis-à-vis the cutting element (2).

8. Cutting device (1) according to one of the previous claims, this also comprising a cutting unit 15 that can separate the epithelial flap (6) completely from the eye.

9. Cutting device (1) according to claim 8, in which the cutting unit comprises a wire (18).

10. Cutting device (1) according to claim 9 with a carriage (9), the wire (18) being movable along the carriage (9).

11. Cutting device (1) according to one of the previous claims, this comprising a suction ring (13) for fixing the cutting device (1) onto the eye.

## Revendications

1. Dispositif de coupe (1) destiné au prélèvement d'un lambeau épithélial (6) d'un oeillet comprenant un corps de coupe (2) pour l'essentiel à symétrie de révolution avec une arête de coupe (11) et un dispositif destiné à la fixation du lambeau épithélial (6) sur un surface d'appui (7), **caractérisé en ce que** le dispositif de coupe présente de plus un espace libre (15) pour le lambeau épithélial (6).

2. Dispositif de coupe (1) selon la revendication 1, dans lequel le dispositif destiné à la fixation du lambeau épithélial (6) comprend un dispositif d'aspiration (14) qui peut générer une dépression entre l'espace libre (15) et le lambeau épithélial (6).

3. Dispositif de coupe (1) selon la revendication 1, dans lequel le dispositif destiné à la fixation du lambeau épithélial (6) comprend un élément réfrigérant (16).

4. Dispositif de coupe (1) selon la revendication 3, dans lequel l'élément réfrigérant (16) est un élément Peltier.

5. Dispositif de coupe (1) selon la revendication 3, dans lequel l'élément réfrigérant (16) est un élément cryogénique.

6. Dispositif de coupe (1) selon l'une quelconque des revendications précédentes, dans lequel la surface d'appui (7) peut être pivotée par rapport au corps de coupe (2).

7. Dispositif de coupe (1) selon l'une quelconque des revendications précédentes, dans lequel la surface d'appui (7) peut être déplacée radialement par rapport au corps de coupe (2).

8. Dispositif de coupe (1) selon l'une quelconque des revendications précédentes, celui-ci comprenant en outre une unité de coupe (15) qui peut séparer complètement le lambeau épithélial (6) de l'oeillet.

9. Dispositif de coupe (1) selon la revendication 8, dans lequel l'unité de coupe comprend un fil (18).

10. Dispositif de coupe (1) selon la revendication 9, avec un chariot (9), le fil (18) pouvant être déplacé le long dudit chariot (9).

11. Dispositif de coupe (1) selon l'une quelconque des revendications précédentes, celui-ci comprenant un anneau d'aspiration (13) destiné à la fixation du dispositif de coupe (1) sur l'oeillet.
